(19)

Europäisches
Patentamt
European
Patent Office
Office européen
des brevets

(11) **EP 2 353 610 A1**

(12) **EUROPEAN PATENT APPLICATION**
published in accordance with Art. 153(4) EPC

(43) Date of publication:
**10.08.2011 Bulletin 2011/32**

(21) Application number: **08878230.5**

(22) Date of filing: **19.11.2008**

(51) Int Cl.:
***A61K 39/145*** (2006.01)

(86) International application number:
**PCT/IB2008/003150**

(87) International publication number:
**WO 2010/058236 (27.05.2010 Gazette 2010/21)**

(84) Designated Contracting States:
**AT BE BG CH CY CZ DE DK EE ES FI FR GB GR HR HU IE IS IT LI LT LU LV MC MT NL NO PL PT RO SE SI SK TR**

(71) Applicant: **Laboratorio Avi-Mex, S.A. De C.V.
México, D.F. 09810 (MX)**

(72) Inventors:
• **LOZANO-DUBERNARD, Bernardo
México, D.F. 04660 (MX)**

• **SARFATI-MIZRAHI, David
Estado De México 52773 (MX)**
• **SUÁREZ-MARTÍNEZ, Jesús Alejandro
México D.F. 06800 (MX)**
• **GAY-GUTIÉRREZ, Manuel Joaquín
México, D.F. 09850 (MX)**
• **SOTO-PRIANTE, Ernesto
México, D.F. 01900 (MX)**

(74) Representative: **Pons Ariño, Angel
Glorieta Ruben Dario 4
28010 Madrid (ES)**

(54) **RECOMBINANT INACTIVATED VIRAL VECTOR VACCINE**

(57) A vaccine is described, comprising an inactivated viral vector having inserted an exogenous nucleotide sequence coding for a disease of concern; and, a pharmaceutically acceptable vehicle, adjuvant or excipient, which provides due protection against the disease of concern by using a viral vector titer similar to that required for an active-virus vaccine based on the same viral vector. Mainly, viral vectors of paramixovirus or adenovirus are described.

**Description**

## TECHNICAL FIELD

The present invention is related to the techniques used in the prevention and treating of diseases, preferably of the avian type, and more particularly, it is related to recombinant vaccines comprising an inactivated viral vector, having inserted an exogenous nucleotide sequence coding for a protein having a disease antigenic activity; and, a pharmaceutically acceptable vehicle, adjuvant or excipient.

## BACKGROUND OF THE INVENTION

As it is well known, vaccines against viral pathogen agents are formulated from the corresponding virus being isolated to be used later in vaccines production, administered to animals or humans through diverse formulations.

On the one hand, there are vaccine formulations using whole and active viruses, having shown low pathogenicity in the field, or with laboratory-attenuated pathogenicity, which however, when administered, cause an antigenic reaction sufficient to provide protection against the same species viral strains having higher pathogenicity.

For example, the Newcastle disease (ND by its English initials) is of viral origin and highly contagious, inclusively it may be lethal. Said disease affects domestic and wild birds, causing high morbidity and mortality. ND is caused by a virus belonging to the *Paramyxoviridae* family, *Avulavirus* genus. According to its pathogenicity and virulence extent, the strains are classified as: lentogenic, mesogenic and velogenic, i.e., low, mild and high pathogenicity, respectively (Office International des Epizooties (2008). Newcastle Disease. OIE Manual of Diagnostic Tests and Vaccines for Terrestrial Animals. Office International des Epizooties. France, p.576-589).

There are multiple transmission sources for the ND virus (NDV). For example, directly through live or dead birds and their products or sub-products, or indirectly through vectors such as infected insects or other animals, including man. The incubation period for the velogenic type NDV (VNDV, by its English initials) causing high mortality, is of about 21 days, showing respiratory and/or nervous signs, such as panting, sneezing and incoordination, bristled wings, leg dragging, twisted head and neck, tics, circle displacement, depression, non-appetency, and complete paralysis. In addition, partial or complete interruption of egg production is shown, or deformed eggs or having thin and rough shell, containing aqueous albumin.

One of the strategies to control and prevent the ND is the use of active-virus vaccines, typically produced from lentogenic strains. Live vaccines against ND induce protection at the respiratory mucosal level, and have been used by industry for more than 50 years. These active-virus vaccines are mainly based on the use of lentogenic viruses from Hitchner B1 and LaSota strains, the latter being the most popular vaccine (Op.Cit., Office International des Epizooties (2008) Newcastle).

However, as active-virus may be inactivated by the components of an emulsion, the stability of emulsified active vaccines is limited. Thus, they are commonly used in other kind of formulations, or, they are delivered by *in situ* mixtures, which difficult its application in large-scale aviculture.

The main problem with active viruses is that they not always can be used as vaccines, due to their high genetic variation ability, recombination with other active viruses, or predisposition to their pathogenicity changes, such as the influenza virus. Influenza is a respiratory disease affecting both mammals and birds. The occurrence of an influenza virus strain in a determined population may have severe consequences for the individuals, for both the domestic birds and for humans or other mammals. When the virus infect domestic hens and mammals, it rapidly mutates to adapt itself to this new population, and during said adaptation evolving process, it may cause important biological changes in the same virus, leading to fatal results for the host and the animal or human population.

Particularly, avian influenza (AI, by its English initials) is a disease having a highly contagious viral etiology caused by a type A virus from the *Orthomyxoviridae* family. Most AI virus (AIV, by its English initials) have been isolated from wild birds, particularly from aquatic birds acting as a reservoir and being carriers of the low pathogenicity AI virus (LPAIV, by its English initials). When these viruses infect a non-natural virus host, such as poultry, mainly *gallinaceae (i.e.,* hens, turkeys and quails, amongst others), the virus suffers mutations to the highly pathogenic form (HPAIV, by its English initials) through an adaptation process.

AIV may be classified according to two virus outer proteins. The first is the hemagglutinin, of the most importance, as it is the responsible of the neutralizing antibody response in infected or vaccinated birds, with 16 different subtypes or serotypes having been reported therefore. The second protein is the neuraminidase, with 9 different subtypes having been reported therefor. Particularly, the most important viruses for birds are those having hemagglutinin serotypes H5 and H7, which when mutating to high pathogenicity, are capable of producing mortalities close to 100%.

Likewise, AI disease in birds shows two clinic forms: the first being low pathogenicity avian influenza (LPAI, by its English initials) causing a mild disease, sometimes expressed as a bad feathers aspect, a decrease in eggs production. But AI is mainly important to birds due to the virus high mutagenic ability, invariably resulting in the second clinic form being the high pathogenicity avian influenza (HPAI, by its English initials) capable of causing mortalities close to 100%.

Particularly, AI clinic signs are variable, and are influenced by the virus subtype involved, its pathogenicity, the immune state and the avian species affected. The incubation period for the HPAIV is of 21 days, and the clinical signs vary from conjunctivitis, temperature rising characterized by feather bristle, depression, prostration and death. The injuries more frequently described are: lung congestion, hemorrhages and edemas.

Once the AIV has been introduced in a poultry farm, this is excreted to the environment through feces and respiratory fluids. The virus transmission and diffusion to other birds is mainly carried out by direct contact with the infected birds secretions, specially contaminated feces, food, water, equipment and clothing. The susceptibility to the infection and the clinic signs manifestation of the disease is highly variable.

For these kinds of diseases, having a difficult control and wherein an active-virus vaccine may involve a risk for the animals and even for the human health in case the control may be lost during its administration, it is preferred using inactivated virus vaccine, typically emulsified.

Several vaccines have been developed in the prior art to prevent diverse viral diseases, such as the above-described AI. Regarding this latter disease, there already exist emulsified vaccines including the AI whole-virus, and which are produced in chicken embryos. This virus is inactivated and emulsified in water-oil for its subcutaneous or intramuscular administration in commercial birds (Office International des Epizooties (2008). Avian Influenza. OIE Manual of Diagnostic Test and Vaccines for Terrestrial Animals, Office International des Epizooties France, p. 465-481).

More particularly, the vaccines made with the AI inactivated virus, stimulate a strong immune response at the systemic level, and they have had positive results to control both AI forms. The vaccination is used both to prevent the clinic signs of the disease, and also to reduce, as possible, the viral excretion from the infected births to the environment. Viral excretion reduction decreases viral dissemination opportunity from vaccinated birds becoming infected, to non-infected susceptible birds (Swayne, D, y Kapczynski, D (2008), Vaccines, Vaccination and Immunology for avian influenza viruses in poultry. In Avian Influenza. Ed by David Swayne. Blackwell Publishing, USA, p.407-451.)

In addition, the emulsified inactivated-virus vaccines have an increased stability, allowing a better vaccine management, and a longer vaccine shelf-life. Therefore, the ND vaccines have also been formulated as emulsified inactivated virus.

It is important considering that one of the main differences between an active-virus vaccine and an inactivated-virus vaccine is the virus amount required to achieve an antigenic response when administered.

Since active viruses have intact their ability to replicate themselves in cells, the amount of the concerned virus required in the vaccine is lower than the dose causing an antigenic response, this to prevent for the individuals being administered with the vaccine to get ill, considering that the virus will naturally replicate and once inside the organism, it will reach enough amounts to achieve the desired antigenic response.

On the other hand, inactivated-virus vaccines require a much higher virus concentration than those of active virus, generally at least 10-fold higher, to achieve the same antigenic activity, since the virus has been manipulated to remove its replication ability, such that the amount of the total antigen required to cause the immune response should be present at the time the vaccine is administered, as the organism will not normally replicate the virus and consequently, its amount will not increase.

On the other hand, one of the most significant advances in the biotechnological field has been the use of recombinant vaccines. The ability to isolate and splice (or to recombine) DNA specific fragments of an organism, with a gene size, and to transfer them to other organism by means of a vector or DNA plasmid to encourage an antigen production capable of inducing the formation of protecting antibodies, has led to the introduction of new vaccines. Contrary to conventional vaccines, recombinant technology provides very important advantages in relation to diseases, such as the AI described above, wherein there is no possibility to use whole active-viruses due to their high mutagenic ability, and wherein the use of the whole inactivated-virus always involves a risk if the inactivation process was inappropriately carried out. Recombinant vaccines, in their active form, as have inserted the necessary nucleotides to express antigens against the disease of concern, may be securely administered to induce local immunity at the respiratory mucosal level in an active viral vector of a low pathogenicity disease, which would be impossible using a non-recombinant live virus due to involved risks.

Another advantage of the recombinant vaccines is that the viral vector employed, typically does not correspond to the disease they protect from, which facilitates their use in the field of veterinary diagnosis and prevention techniques of the type allowing to differentiate vaccinated animals from infected animals, better known as DIVA (Capua, I et al., "Development of a DIVA (differentiating infected from vaccinated animals) strategy using a vaccine containing a heterologous neuraminidase for the control of avian influenza". Avian Pathology 32(1) pp. 47-55).

Now then, vaccines currently used to control AI (emulsified in oil, whole inactivated -virus) and other similar diseases, prevent the mortality caused by the HPAIV, but do not avoid the infection and replication of the AIV in birds, therefore, the decrease in excretion and virus dissemination is partially achieved.

Therefore, viral vectors have been developed in the prior art from low pathogenicity diseases, such as Newcastle, having inserted genes coding for antigenic sites of difficult control diseases, such as avian influenza. Such is the case of the document Ge, Deng, Tian et al. "Newcastle disease virus-based live attenuated vaccine completely protects chickens and mice", J. Vir. Vol. 81, No. 1, p. 150-158", which discloses a recombinant vaccine in active form. Particularly, said document discloses the result from clinical trials using the LaSota strain having an avian influenza subtype H5N1 gene.

Another prior art document, from the same field is Park, Man Seong et al. "Engineered viral vaccine constructs with dual specificity: Avian Influenza and Newcastle disease". PNAS Vol. 103, No. 21, May 12, 2006 p. 8203-8208. Said document is related to a technology increasing avian influenza gene expression, such technology hereinafter is called "anchoring".

Although some recombinant vaccines have replaced active-virus vaccines due to the above-mentioned advantages, recombinant vaccines have not reached yet the advantages of the inactivated whole-virus vaccines, and above all, they have not been able to provide the proper immunity with respect to the inserted exogenous gene, mainly due to the fact that recombinant vaccines, such as the above-described Newcastle with influenza, cause antigenic activity against both diseases, but require a higher exposure of the exogenous antigenic sites being inserted in the vector. As a consequence, technologies development has been sought, such as the anchoring, which by means of genetic modifications, as in the case of influenza above described, yield a better antigen expression in the viral vector. However, such technologies have not been entirely successful.

Thus, recombinant vaccines from active virus typically are formulated with a virus concentration of about 10-fold higher than that used for the non-recombinant vaccine from active virus, corresponding to the viral vector being used, with the purpose of achieving a suitable exposure of the antigenic sites of the microorganism of concern.

Likewise, recombinant vaccines have not been used in the inactivated form, since that would imply achieving viral vector concentrations 100-fold higher than those required for the normal virus (10-fold higher than the recombinant active virus), which would be very complicated at the industrial level. Consequently, in general, these recombinant active-virus vaccines have neither been used as emulsions, due to a limited stability and because the emulsion is not advantageous in this respect due to the active nature of the active viral vector.

Summarizing, it can be seen from the above that there is a great need for vaccines against diverse diseases by recombinant technology, in a safer and efficient manner, such that a better stability is achieved in the produced vaccines, with appropriate control and efficacy results.

## BRIEF DESCRIPTION OF THE INVENTION

While developing the present invention, it was unexpectedly found that a vaccine comprising a recombinant inactivated viral vector, having inserted an exogenous nucleotide sequence coding for an antigenic site of a disease of concern; and, a pharmaceutically acceptable emulsified vehicle, adjuvant or excipient, provides due protection against said disease of concern by using a viral vector titer similar to that required for a recombinant active-virus vaccine based on the same viral vector.

In an embodiment of the invention, the exogenous nucleotide sequence is selected from antigenic sites sequences against influenza, infectious laryngotracheitis, infectious bronchitis, bursa of Fabricius' infection (Gumboro), hepatitis, viral rhinotracheitis, infectious coryza, *Mycoplasma hyopneumonieae,* pasteurellosis, Porcine Respiratory and Reproductive Syndrome (PRRS), circovirus, bordetellosis, parainfluenza, or any other antigen which size allows its insertion into the corresponding viral vector. Preferably, an antigen selected from avian influenza, laryngotracheitis, infectious bronchitis, bursa of Fabricius' infection (Gumboro), hepatitis, PRRS, and circovirus, is used.

In an specific embodiment of the present invention, the exogenous nucleotide sequence consists of the gene coding for hemagglutinin (HA) of the avian influenza virus, selected from the hemagglutinin 16 subtypes or immunogenic variant of the influenza virus, which more preferably codifies to at least one of subtypes H1, H2, H3, H5, H6, H7 or H9 of said protein.

In an specific embodiment of the invention, the protein H5-gene is obtained from the Mexican avian influenza virus subtype H5N2, or from the Asian-originated subtype H5N1, observing excellent protection of both subtypes against mortality induced by HPAIV subtype H5N2.

Regarding the viral vector of the present invention, in the preferred embodiment wherein the Newcastle disease virus (rNDV) corresponds to the viral vector having inserted the exogenous nucleotide sequence, said viral vector is preferably selected from vaccinal strains, such as LaSota, Ulster, QV4, B1, CA 2002, Roakin, Komarov, Clone 30, or VGGA strains, or strains from the Newcastle disease genetic groups I to V. Preferably, the recombinant virus is of LaSota strain (rNDV/LS).

Likewise, in a further specific embodiment wherein the viral vector is an adenovirus, the adenovirus is selected from avian and porcine adenoviruses, and more preferably, from the avian adenovirus type 9 (rFAdV/9) and porcine adenovirus type 5 (rSAdV/5).

According to the obtained results detailed below, it is concluded that by means of the present invention it is possible to use an exogenous nucleotide sequence coding for specific antigenic determinants of a disease of concern, in a viral vector to produce a recombinant inactivated-virus vaccine in an emulsion or in other pharmaceutically acceptable adjuvants.

The result achieved with the vaccine of the present invention (rNDV/LS-H5) is unexpected, since traditionally it has been believed that in the case of recombinant vaccines in viral vector, the viral vector replication in the host cells is required to achieve enough recombinant protein expression to stimulate a suitable immunogenic response, however, in the present invention, the obtained result shows that the antigenic protein of the disease of concern is enough and properly expressed in the vector virus surface, and its only presence in the inactive form enables a suitable antigenic and protective response against said disease of concern.

Particularly, in the case of high pathogenicity and difficult to control diseases, such as avian influenza, an advantage of the recombinant vaccine of the present invention is that the whole virus is not used, thereby suppressing the risk of an outbreak from an inappropriate inactivation of the vaccinal virus. Moreover, the vaccine of the present invention achieves a local immune response at the bird's respiratory mucosa level, as well as an immune response at the systemic level, capable of being differentiated through specific laboratory tests, from immune responses induced by the birds' contact with whole viruses, either vaccinal or field-type, representing an important advance in the epidemiologic field.

The vaccine is formulated to be subcutaneously administered; however, any systemic route such as intramuscular or intradermal may be successfully used. A liquid vehicle for the vaccine is preferably used, more preferably, a water-in-oil emulsion is used, but it is also successful to use other kind of immune response adjuvants or modulators.

With the recombinant vaccine of the present invention, decreases the excretion of the field-type virus to the environment, thereby contributing to greatly reduce the virus spreading.

## BRIEF DESCRIPTION OF THE FIGURES

The novel aspects considered characteristics of the present invention, will be particularly set forth in the appended claims. However, the vaccine of the present invention, together with other objects and advantages thereof, will be better understood from the following detailed description of certain specific embodiments, when read with relation to the appended drawings, wherein:

Figure 1 is a plot of the mortality results (M) and the morbidity index (MI) from Example 6A, produced by the challenge with a velogenic NDV (VNDV).

Figure 2 is a plot of the mortality results (M) and the morbidity index (MI) from Example 6A, produced by the challenge with a high pathogenicity AI virus (HPAIV) subtype H5N2.

Figure 3 is a plot of the mortality results (M) and the morbidity index (MI) from Example 6B, produced by the challenge with a VNDV.

Figure 4 is a plot of the mortality results (M) and the morbidity index (MI) from Example 6B, produced by the challenge with a HPAIV subtype H5N2.

Figure 5 is a plot of the mortality results (M) and the morbidity index (MI) from Example 6C, produced by the challenge with a VNDV.

Figure 6 is a plot of the mortality results (M) and the morbidity index (MI) from Example 6C, produced by the challenge with a HPAIV subtype H5N2.

Figure 7 is a plot of the mortality results (M) and the morbidity index (MI) from Example 6D, produced by the challenge with a VNDV.

Figure 8 is a plot of the mortality results (M) and the morbidity index (MI) from Example 6D, produced by the challenge with a HPAIV subtype H5N2.

## DETAILED DESCRIPTION OF THE INVENTION

While developing the present invention, it was unexpectedly verified that a vaccine comprising an inactivated viral vector, having inserted a nucleotide sequence coding for a disease of concern; and, a pharmaceutically acceptable vehicle, adjuvant or excipient, provides due protection against the disease of concern by the use of a viral vector titer similar to that required for an active-virus vaccine based on the same viral vector.

In the present invention is essential for the viral vector to be inactivated, inactivated meaning that the recombinant virus acting as a viral vector and containing the nucleotide sequence coding for the antigenic site of the disease of concern, has lost the replication property. The inactivation is achieved by physical or chemical procedures well known in the state of the art, preferably by chemical inactivation with formaldehyde or beta-propiolactone (Office International des Epizooties (2008). Newcastle Disease. OIE Manual of Diagnostic Tests and Vaccines for Terrestrial Animals. Office International des Epizooties. France, p. 576-589). On the contrary, a live or active virus means keeping its replication ability.

The viral vector, preferably selected from adenovirus or paramixovirus, is inactivated and has inserted an exogenous nucleotide sequence coding for at least one antigenic site of a disease of concern, preferably of at least one disease selected from influenza, infectious laryngotracheitis, infectious bronchitis, bursa of Fabricius' infection (Gumboro), hepatitis, viral rhinotracheitis, infectious coryza, *Mycoplasma hyopneumoniae,* pasteurellosis, Porcine Respiratory and Reproductive Syndrome (PRRS), circovirus, bordetellosis, parainfluenza or any other antigen which size allows its insertion into the corresponding viral vector. More preferably, an antigen selected from avian influenza, laryngotracheitis, infectious bronchitis, bursa of Fabricius' infection (Gumboro), hepatitis, PRRS, and circovirus, is used.

In an specific embodiment of the present invention, the exogenous nucleotide sequence consists of the hemagglutinin (HA)-coding gene of the avian influenza virus, selected from the hemagglutinin 16 subtypes or immunogenic variant of the influenza virus, which more preferably codifies for at least one of subtypes H1, H2, H3, H5, H6, H7 or H9 of said protein.

Regarding the viral vector of the present invention, in the preferred embodiment wherein the Newcastle disease virus (rNDV) corresponds to the viral vector wherein the exogenous nucleotide sequence is inserted, said viral vector is preferably selected from vaccinal strains, such as LaSota, Ulster, QV4, B1, CA 2002, Roakin, Komarov, Clone 30, VGGA strains, or strains from the Newcastle disease genetic groups I to V. Preferably, the recombinant virus is of LaSota strain (rNDV/LS).

Likewise, in a further embodiment wherein the viral vector is an adenovirus, the adenovirus is selected from avian and porcine adenovirus, and more preferably, from the avian adenovirus type 9 (rFAdV/9) and porcine adenovirus type 5 (rSAdV/5).

Regarding the antigenic site, when influenza is the disease of concern, the antigenic site corresponding to the avian influenza hemagglutinin (HA) protein is preferred, preferably obtaining the gene from the avian influenza virus, and coding for any of the existing 16 subtypes, preferably H5, H7 and H9, preferably coding for subtype H5, which preferably is obtained from: Bive, 435 and Viet (VT) strains, described below. In this regard, it may be inferred that the gene-source strain coding for HA subtype H5, is not critical for the present invention since the experimental results show that any strain can provide the genetic material useful to achieve the goal of the present invention.

Regarding the preferred gene-sources, it is worth mentioning that the H5-gene from Bive strain corresponds to a LPAIV-H5N2 isolated in Mexico in 1994 from broilers' biological samples, and having been identified by the Mexican government as (A/chicken/Mexico/232/CPA). Said virus strain is authorized by the "Secretaria de Agricultura, Ganaderia, Desarrollo Rural, Pesca y Alimentación (SAGARPA, by its Spanish acronym)" for its use in the manufacture of emulsified inactivated vaccines, thus, the recombination of this virus with the gene of concern also ensures a biosafety in the recombinant vaccine of the present invention.

Regarding the second preferred genetic material source being H5-435-gene, this was obtained from the isolation of a LPAIV-H5N2, isolated in Mexico in 2005 from broilers' biological samples.

The viral vector of the vaccine of the present invention can be prepared by a PCR amplification of the nucleotide sequence of interest, by identifying the antigenic sites from an isolation of the origin-pathogen, to be further inserted, amplified in the viral vector, preferably selected from adenovirus or paramixovirus. The insertion is made using standard molecular biology techniques, such as restriction enzymes and DNA ligases, among others. The infectious clone thus produced is introduced into a cell line for the production of the recombinant virus according to the viral vector.

Depending on the nature of the viral vector, the virus replicates in any suitable growing system, such as SFP chicken embryos, or commercial cell lines, or specifically designed for the virus growing.

Once the virus concentration required for achieving the antigenic response is reached, preferably between $10^2$ and $10^{10}$ DI50%/ml, depending on the viral vector used, the virus inactivation proceeds. Preferably, the inactivation is carried out by physical or chemical procedures well known in the state of the art, preferably by chemical inactivation with formaldehyde or beta-propiolactone.

Pharmaceutically acceptable vehicles for the vaccines of the present invention are preferably aqueous solutions or emulsions. More particularly, the use of a water-in-oil emulsion vehicle is preferred. The vaccine specific formulation will depend on the viral vector used, as well as on the exogenous nucleotide sequence having been inserted. However, in the preferred embodiment wherein the viral vector is a Newcastle disease virus, the preferred dose is between $10^4$ and $10^{10}$ DIEP50%/ml. In the embodiment wherein an adenovirus is the viral vector, the preferred dose is between $10^2$ and $10^8$ DIEP50%/ml.

Regarding the vaccine administration, this is preferably administered by subcutaneous route in the rear middle portion of the bird's neck. The vaccine of the present invention is administered to poultry, such as broilers, laying birds, reproduction birds, turkeys, fighting cocks, Guinea fowls, partridges, quails, ducks, gooses, swans or ostriches. Preferably, the vaccine is subcutaneously administered, although in certain species it may be intramuscularly administered in birds of any age.

When the vaccine is applied in chicken in emulsified Newcastle vector, the vaccine preferably contains $10^8$ to $10^9$ DIEP50%/0.5ml per chicken, and more preferably, the vaccine contains $10^{8.5}$ DIEP50%/0.5ml per chicken. Chicken vaccination may be easily made at 10 days-old.

The present invention provides very important competitive advantages. The recombinant inactive vaccine of the present invention makes possible to establish vaccination programs exclusively using recombinant vaccines in viral vector and with the insertion of genes from pathogenic agents difficult to control, which results in an identification method of infected animals from animals having received only one vaccine (DIVA), useful in the disease control and eradication, comprising:

a) subjecting to a first antibodies detection method, at least one sample of at least one animal having received a recombinant vaccine of inactivated viral vector having inserted an exogenous nucleotide sequence coding for an antigen of a disease caused by a pathogen, to detect if there are antibodies present in said sample corresponding to said antigen;
b) subjecting to a second antibodies detection method, at least one sample from the same animal which sample was subjected to the first antibodies detection method, to detect if there are antibodies present in said sample corresponding to the pathogen causing the disease;
c) determining if the animal is infected or vaccinated from the results of the first and second antigens detection methods.

For example, when the pathogen is difficult to control, such as the AIV, mainly H5 and H7, causing high mortality in poultry, with the recombinant inactivated vaccine of the present invention, an excellent systemic level protection is achieved, offering also a biosafety high degree compared to the use of AI whole virus constituting a severe risk in case of not having been properly inactivated. This risk increases during the manufacturing process wherein the viruses are active. The present invention also permits the epidemiologic differentiation of vaccinated birds from other birds exposed to whole viruses (DIVA system), since when only the avian influenza virus hemagglutinin (HA)-gene is inserted, the laboratory test used to detect the vaccine-induced antibodies against the avian influenza is the hemagglutination inhibition (HI). Current immunological tests, such as ELISA and other tests as agar gel diffusion, are negative to the antibodies detection against the avian influenza induced by the recombinant vaccine of the present invention, as they are designed to detect antibodies induced by other kind of antigens contained in the whole viruses. When birds vaccinated with the recombinant vaccine of the present invention are infected with the field-type virus, these tests are positive to antibodies detection against avian influenza, thereby the infected birds may be distinguished.

Additionally, the present invention allows establishing joint programs exclusively using recombinant vaccines in an inactive and active form, the first will give the above-mentioned systemic immunity, and the recombinant active vaccine will complement the immunity at the mucosa level, yielding protections equal or close to 100% at a field level. With this program the above-mentioned DIVA system is also used.

In the preferred embodiment of the invention wherein the recombinant vector of the emulsified inactivated vaccine is Newcastle with an influenza-gene inserted, for both challenges with VNDV and HPAIV, this may be simultaneously administered with an active vaccine with the same vector and antigen, directly to the respiratory mucosa, either by ocular route, by spraying, or in drinking water, such that the local level response be highly stimulated (in the respiratory and digestive mucosa) producing secretory immunoglobulins type A (IgA), thereby significantly decreasing the field-type virus replication, thus significantly reducing its excretion and spreading.

On the other hand, the vaccine of the present invention permits establishing control programs and possible eradication by differentiating vaccinated from infected birds, since it is possible, when administering the recombinant inactivated vaccines of the present invention, to differentiate vaccinated birds from infected birds with the field-type virus (DIVA system), since recombinant vaccines only contain the AIV hemagglutinin as antigen, allowing the use of diagnostic tests such as ELISA, which detects antibodies induced by other virus' antigens, and not only those induced by hemagglutinin.

The recombinant vaccine against influenza of the present invention will be more clearly illustrated by means of the following description of specific examples, which are provided only with illustrative purposes, and not to limit the invention.

## EXAMPLES

## EXAMPLE 1

### Production of the Newcastle-LaSota vector

In order to clone the Newcastle-virus LaSota-strain genome and thereby producing a viral vector, firstly, an intermediate vector called "pNDV/LS" was produced. A total viral RNA extraction was carried out for the Newcastle-LaSota strain by the triazole method. The cDNA (complementary DNA) synthesis was made from the purified RNA of the viral genome, using the previously purified total RNA as a template. With the purpose of cloning all genes from the Newcastle genome (15, 183 base pairs (bp)), 7 fragments having "overlapping" ends and cohesive restriction sites were amplified by PCR. Fragment 1 (F1) comprises from nucleotide (nt) 1-1755, F2 from nt 1-3321, F3 comprises from nt 1755-6580, F4 from 6,151-10,210, F5 comprises from nt 7,381-11,351, F6 from 11,351-14,995 and F7 comprises from nt 14,701-15,186. The assembly of the 7 fragments was carried out in a cloning vector called pGEM-T using standard linking techniques, thereby rebuilding the Newcastle-LaSota genome, which after the cloning has a single restriction site Sacll, between P and M genes, serving to clone any gene of concern in this vector viral region.

**EXAMPLE 2**

**HA-gene cloning from AIV subtype H5N2 435 strain 435**

Total viral RNA extraction was carried out to clone the HA-gene of AIV 435 strain by the Triazole method. This purified total RNA was used later to synthesize cDNA (complementary DNA), and by the PCR technique, the HA-gene from AI virus was amplified using specific oligonucleotides. The HA gene from 435 was then inserted into the pGEM-T vector, using standard cloning techniques and producing the plasmid: p-GEMT-435.

**EXAMPLE 3**

**HA-gene cloning of AI 435 within SacII site of pNDV/LS vector to produce plasmid: pNDV/LS-435**

A. Intermediate pSacIIGE/GS vector production:

A new intermediate vector called pSacIIGE/GS was built to introduce transcription sequences from Newcastle called GE/GS at 5' end of the HA 435 gene, by the PCR initial amplification of sequences GE/GS, taking the Newcastle genome as a template and the later insertion of these sequences in pGEM-T.

B. HA-gene subcloning to pSacIIGE/GS vector:

Plasmid pGEMT-435 was digested with Hpal-Ndel and further cloned into pSacIIGE/GS, to produce plasmid pSacIIGE/GS-HA435.

C. Subcloning of GE/GS-HA435 to pNDV-LS vector

Both plasmids: pSacIIGE/GS-HA435 and pNDV/LS, were digested with Sacll, the digestion products were purified and the GE/GS-HA435 region was purified and inserted into Sacll site of pNDV/LS, thereby producing the infective clone called: pNDV/LS-435.

**EXAMPLE 4**

**Production of recombinant virus rNDV/LS-HA435 in cell culture**

Hep-2 and A-549 cells were initially infected with MAV-7 virus at an infection multiplicity (MOI) of 1. After incubation for 1 hour at 37°C in 5% $CO_2$ atmosphere, cells were transfected with 1 microgram ($\mu$g) DNA of clone pNDVLS-435, together with 0.2 $\mu$g DNA from expression plasmids: pNP, pP and pL, which codify for viral proteins P, NP and L, needed to produce the recombinant in both cell types. 12 hours after transfection, the recombinant virus produced in both cell types was harvested and injected to 10 days-old SPF chicken embryos to amplify the produced virus. Allantoid liquid harvested 48 hours later, was titrated by plate assay in Vero cells, thereby producing the final recombinant virus, used to prepare the vaccine.
Recombinant virus having the genes obtained from Bive and Viet strains were produced as described above.

**EXAMPLE 5**

**Manufacturing method for emulsified inactivated vaccine with Newcastle-LaSota recombinant virus having H5 insert from avian influenza virus: rNDV/LS-H5**

Antigen production

Starting from the production seed, chicken embryonated eggs, free from specific pathogens (SPF), were inoculated with the previously determined infecting dose. Embryos were incubated at 37°C for 72 hours, checking mortality daily. After this period, live embryos were refrigerated from one day to the next day, preferably for 24 hours, and the amnioallantoid fluid (FAA, by its Spanish acronym) was harvested aseptically. The FAA was clarified by centrifugation and was inactivated with formaldehyde, although any other inactivating agent known may be used, commonly used in the production of this kind of vaccines. The FAA was subjected to tests confirming its inactivation, purity, sterility and titer for both DIEP and HA.

Emulsion production

Vaccine was prepared in a water-in-oil type emulsion. Mineral oil and surfactants type Span 80 and Tween 80 were used in the oily phase. To prepare the aqueous phase, the FAA was mixed with a preservative solution (thimerosal).To prepare the emulsion, the aqueous phase was added slowly to the oily phase with constant stirring. A homogenizer or colloidal mill was used to reach the specified particle size.

Antigenic content

Vaccine was formulated to give a minimum of $10^{8.5}$ DIEP50%/0.5ml, in order to use a dose of 0.5ml per bird.
Based on the above procedure, six recombinant vaccines were produced: three having Newcastle-LaSota strain vector (rNDV/LS) with HA anchoring for AI virus, called (Rd), and three more having the same vector but without anchoring, called (Re); Rd and Re genome each was cloned with three different HA-genes indicated below to obtain the 6 vaccines:

1. H5-Bive gene: Obtained from LPAIV subtype H5N2 strain (A/chicken/Mexico/232/CPA), isolated in Mexico in 1994 from broilers' biological samples, and corresponding to the virus strain authorized by the SAGARPA to produce emulsified inactivated vaccines.
2. H5-435 gene: Obtained from an isolation of HPAIV subtype H5N2, isolated in Mexico in 2005 from broilers' biological samples. 435 strain showed differential antigenic features in hemagglutinin inhibition (HI) tests with the Bive strain, and important changes in nucleotide sequencing.
3. H5-Vt gene: This gene was isolated in Vietnam and corresponds to H5-gene of an AI virus subtype H5N1.

## EXAMPLE 5A

An emulsified, inactivated, recombinant, with anchoring (Rd), and H5-Bive gene, in (rNDV/LS) vector, experimental vaccine was produced according to the method described in example 5, as a water/oil pharmaceutical formula, which was called Emi Rd-Bive.

## EXAMPLE 5B

An emulsified, inactivated, recombinant, with anchoring (Rd), and H5-435 gene, in (rNDV/LS) vector, experimental vaccine was produced according to the method described in example 5, as a water/oil pharmaceutical formula, which was called Emi Rd-435.

## EXAMPLE 5C

An emulsified, inactivated, recombinant, with anchoring and H5-Vt gene, in (rNDV/LS) vector, experimental vaccine was produced according to the method described in example 5, as a water/oil pharmaceutical formula, which was called Emi Rd-Vt.

## EXAMPLE 5D

An emulsified, inactivated, recombinant, with no anchoring and H5-Bive gene, in (rNDV/LS) vector, experimental vaccine was produced according to the method described in example 5, as a water/oil pharmaceutical formula, which was called Emi Re-Bive.

## EXAMPLE 5E

An emulsified, inactivated, recombinant, with no anchoring and H5-435 gene, in (rNDV/LS) vector, experimental vaccine was produced according to the method described in example 5, as a water/oil pharmaceutical formula, which was called Emi Re-435.

## EXAMPLE 5F

An emulsified, inactivated, recombinant, with no anchoring and H5-Vt gene, in (rNDV/LS) vector, experimental vaccine was produced according to the method described in example 5, as a water/oil pharmaceutical formula, which was called Emi Re-Vt.

## EXAMPLE 6

## Potency *in vivo* assessment for recombinant vaccines in ND-LaSota vector with and without anchoring for AI virus HA-gene

In order to determine the efficacy of the emulsified recombinant inactivated vaccines of the present invention was determined, and demonstrate that these may be produced with different cloned hemagglutinin genes from different antigenic subtypes and variants of AI virus, their efficacy to prevent mortality caused by HPAI virus subtype H5N2 and VNDV in SPF birds and, on the other hand, in commercial broilers having parental immunity to AIV and NDV, was tested.
The strains used in the different challenging experiments to measure the vaccines efficacy were as follows:

1. Avian Influenza **(HPAIV-H5N2):** High pathogenicity virus subtype H5N2, A/chicken/Querétaro/14588-19/95 strain with titer of $10^{8.0}$ DIEP50%/ml, equivalent to 100 DLP50%/0.3ml/chicken.
2. **VNDV** virus: Chimalhuacan strain containing $10^{8.0}$ DIEP50%/ml, equivalent to $10^{6.5}$ DIEP50%/0.03ml/chicken.

Challenges were made in 35 days-old chicken (21 days post-vaccination -DPV-) in isolation units at INIFAP-CENID-Microbiology, in acrylic isolation cabinets having a biosafety level 3. For the challenges, each experimental group was divided in two subgroups, and each subgroup was assigned at the corresponding isolation units according to the pre-established biosafety procedures.
HPAIV-H5N2 was diluted at a 1:10 ratio with PBS at pH 7.2, and 0.06 ml (2 drops) was administered to each chicken at each eye, and 0.09 ml (3 drops) at each nostril, equivalent to 0.3 ml or 100 DLP50%.
The VNDV virus challenge was made administering by ocular route to each chicken, 0.03 ml of a viral suspension containing $10^{8.5}$ DIEP50%/ml, equivalent to $10^{6.5}$ DLP50%/bird.
For the post-challenge (PC) assessment, all groups were daily checked to record mortality and morbidity, including clinical signs severity, monitoring the birds individually in each group every PC day (DPC), assigning them a numeric value according to the criterion in Table 1:

TABLE 1-Values for mortality and morbidity records

| Clinical Signs | Mild | Severe |
|---|---|---|
| Without apparent clinical signs | 0 | |
| Conjunctivitis | 1 | 2 |
| Conjunctivitis + bristled feathers | 3 | 4 |
| Conjunctivitis + bristled feathers + prostration | 5 | 6 |
| Death | 7 | |

PC assessment with VNDV was carried out for 14 days, while PC assessment with HPAIV-H5N2 was carried out for 10 days according to the guidelines suggested by the OIE.
Morbidity index (MI) for each group was calculated by an equation obtained from the data corresponding to the more severe clinic signology day during the observation period PD.

$$MI = \frac{(A)\,(100)}{B}$$

Wherein

A = the sum of all individual values for injury severity at the observation day
B = maximum possible severity value of the clinical condition in one day.

The experiments as carried out are described below.

## EXAMPLE 6A

Challenges were made with a VNDV and HPAIV-H5N2 at 21 DPV in SPF birds groups, which were immunized, as indicated in Table 2, with the inactivated vaccines of the present invention obtained according to Examples 5A (Emi Rd-Bive), 5B (Emi Rd-435) and 5C (Emi Rd-Vt). For comparative purposes, two other groups were immunized with two emulsified commercial vaccines against avian influenza and Newcastle disease, produced from emulsified inactivated whole-virus, called E. ND/AI-435 and E. ND/AI-Bive, respectively.

TABLE 2- Potency in SPF birds immunized with inactivated vaccines produced with the recombinant virus rNDV/LS-H5 with anchoring (Rd)

| Group | No. of birds | Administered vaccines at 14 days-old by SC route | SPF birds number per challenge strain administered at 21 DPV | |
| --- | --- | --- | --- | --- |
| | | | VAIAP-H5N2 | VVENV |
| 1 | 50 | Emi Rd-Bive | 25 | 25 |
| 2 | 50 | Emi Rd-435 | 25 | 25 |
| 3 | 50 | Emi Rd-Vt | 25 | 25 |
| 4 | 50 | E. ND/AI-435 | 25 | 25 |
| 5 | 50 | E. ND/AI-Bive | 25 | 25 |
| 6 | 50 | Positive control | 25 | 25 |
| 7 | 20 | Negative control | ---- | ---- |

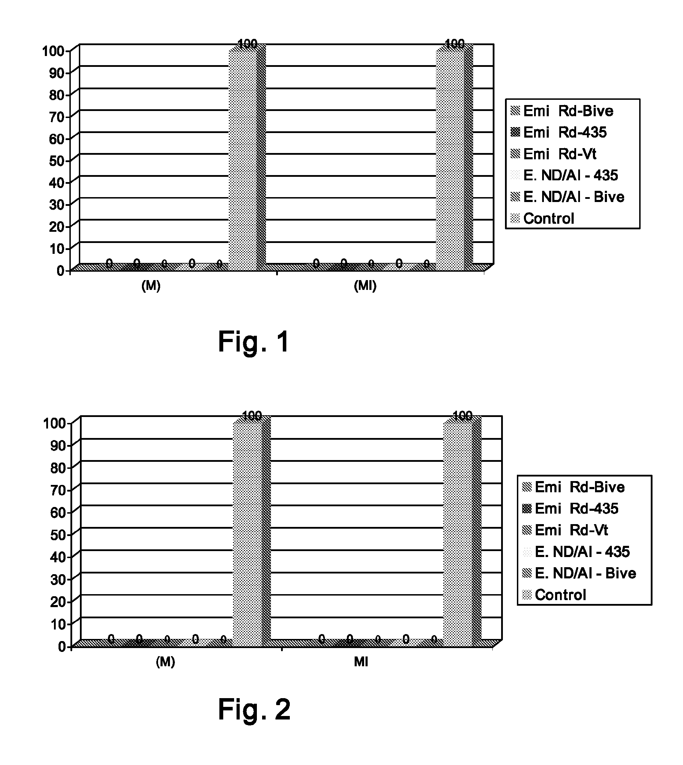

Potency results against VNDV and HPAIV-H5N2 are graphically shown in Figures 1 and 2, respectively.
Results show that all three recombinant inactivated vaccines rNDV/LS-H5 with anchoring of the present invention are capable to provide in SPF chicken 100% protection against mortality (M) induced by the VNDV challenge virus. Likewise, and regardless the H5-gene with which they were cloned, all three recombinant inactivated vaccines also provide 100% protection against mortality (M) induced by HPAIV-H5N2 (Figure 2) equally to the conventional inactivated vaccines produced with whole-virus currently authorized worldwide to be used in the control of ND and AI, which are produced typically including in the formula virus of Newcastle disease LaSota strain with a titer of $10^{8.6}$ DIEP50%/ml, and low pathogenicity avian influenza virus with a titer of $10^{8.0}$ DIEP50%/ml, chemically inactivated with formaldehyde and oil-emulsified. The protection results show that the recombinant inactivated vaccines rNDV/LS-H5 with anchoring meets the Mexican and International Standards for its use to control ND and AI, then, this recombinant version with anchoring of the present invention proved to be successful.

## EXAMPLE 6B

As a second experimental design to determine the anchoring effect, challenges were made with VNDV and HPAIV-H5N2 at 21 DPV in groups of SPF birds, which were immunized, as shown in Table 3, with two commercial emulsified vaccines against avian influenza and Newcastle disease, produced with emulsified inactivated whole-virus, called **E. ND/AI-435** and **E. ND/AI-Bive,** as well as with three emulsified inactivated vaccines of the present invention, without anchoring, obtained from Examples 5D **(Emi-ReBive),** 5E **(Emi-Re-435)** and 5F **(Emi-Re-Vt).**

TABLE 3- Potency in SPF birds immunized with inactivated vaccines produced with the recombinant virus rNDV/LS-H5 without anchoring (Re)

| Group | No. of birds | Administered vaccines at 14 days-old by SC route | SPF birds number per challenge strain administered at 21 DPV | |
|---|---|---|---|---|
| | | | HPAIV-H5N2 | VVNDV |
| 1 | 50 | Emi Re-Bive | 25 | 25 |
| 2 | 50 | Emi Re-435 | 25 | 25 |
| 3 | 50 | Emi Re-Vt | 25 | 25 |
| 4 | 50 | E. ND/AI-435 | 25 | 25 |
| 5 | 50 | E. ND/AI-Bive | 25 | 25 |
| 6 | 50 | Positive control | 25 | 25 |
| 7 | 20 | Negative control | ---- | ---- |

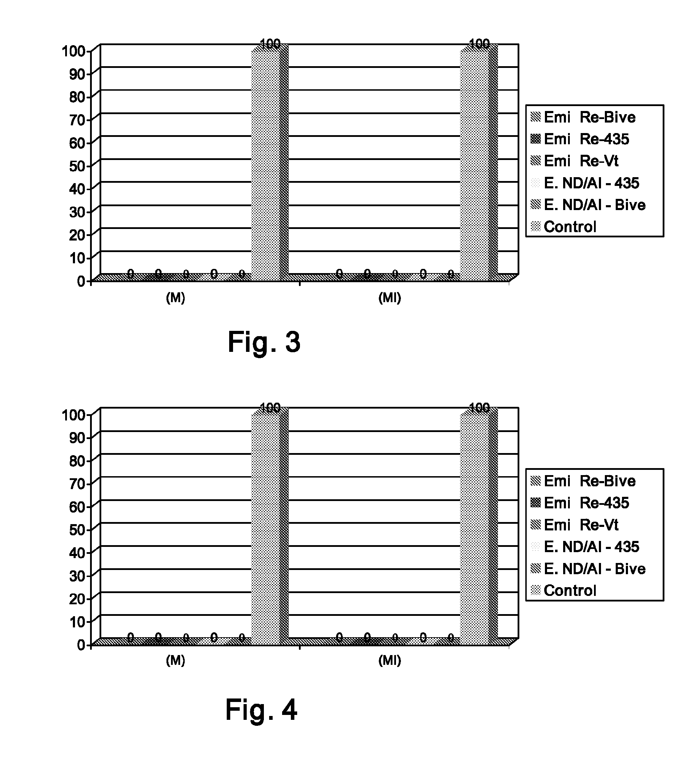

Potency results against VNDV and HPAIV-H5N2 are graphically shown in Figures 3 and 4, respectively.

Unexpectedly, the results show that all three recombinant inactivated vaccines rNDV/LS-H5 without anchoring of the present invention are also capable of providing in SPF birds 100% protection against mortality (M) induced by the VNDV challenge virus. Likewise, and regardless H5-gene with which they were cloned, all three recombinant inactivated vaccines also provided 100% protection against mortality (M) induced by HPAIV-H5N2, equally to the recombinant inactivated vaccines rNDV/LS-H5 with anchoring, and conventional emulsified vaccines produced with inactivated whole-virus ND/AI-Bive and ND/AI-435.

The results from Examples 6A and 6B show that the recombinant inactivated vaccines produced in vector with or without anchoring, and with AIV H5-genes from different origin and antigenic characteristics in HI tests (H5N2 or H5N1), are capable of providing the same protection to the challenge with HPAIV-H5N2. Results suggests that the recombinant inactivated vaccines produced with any AIV H5-gene may provide protection against the HPAIV challenge with any one of the influenza virus subtypes having hemagglutinin H5, the kind of neuraminidase not being relevant.

Therefore, it has been shown that the present invention is effective inclusive to different types of neuraminidase, which is consistent to that found for traditional inactivated whole-virus vaccines (Soto et al., Inactivated mexican H5N2 avian influenza vaccine protects chickens from the asiatic highly pathogenic H5N1 avian influenza virus. Proceedings of the 56th Western Poultry Disease Conference (WPDC). USA, p. 79. (2007) and Swayne, D. and Kapczynski, D. (2008). Vaccines, Vaccination and Immunology for avian influenza viruses in poultry. In Avian Influenza. Ed. By David Swayne. Blackwell Publishing, USA, p. 407-451).

## EXAMPLE 6C

A third experiment was carried out in order to test the vaccines of the present invention in commercial birds to simulate field conditions, wherein challenges were made using a VNDV and a HPAIV-H5N2 at 21 DPV in commercial broilers with parental immunity to ND and AI, which were immunized as indicated in Table 4, with two emulsified commercial vaccines against avian influenza and Newcastle disease, produced with emulsified inactivated whole-virus called **E. ND/AI-435,** and **E. ND/AI-Bive,** as well as the inactivated vaccines of the present invention obtained according to Examples 5A **(Emi-Rd-Bive),** 5B **(Emi-Rd-435)** and 5C **(Emi-Rd-Vt).**

TABLE 4 -Potency in commercial broilers with parental immunity to ND and AI, immunized with inactivated vaccines produced with the recombinant virus rNDV/LS-H5 with anchoring (Rd)

| Group | No. of chickens | Administered vaccines at 14 days-old by SC route | Number of commercial broilers per challenge strain administered at 21 DPV | |
|---|---|---|---|---|
| | | | VAIAP-H5N2 | VVNDV |
| 1 | 50 | Emi Re-Bive | 25 | 25 |
| 2 | 50 | Emi Re-435 | 25 | 25 |
| 3 | 50 | Emi Re-Vt | 25 | 25 |
| 4 | 50 | E. ND/AI-435 | 25 | 25 |
| 5 | 50 | E. ND/AI-Bive | 25 | 25 |
| 6 | 50 | Positive control | 25 | 25 |
| 7 | 20 | Negative control | ---- | ---- |

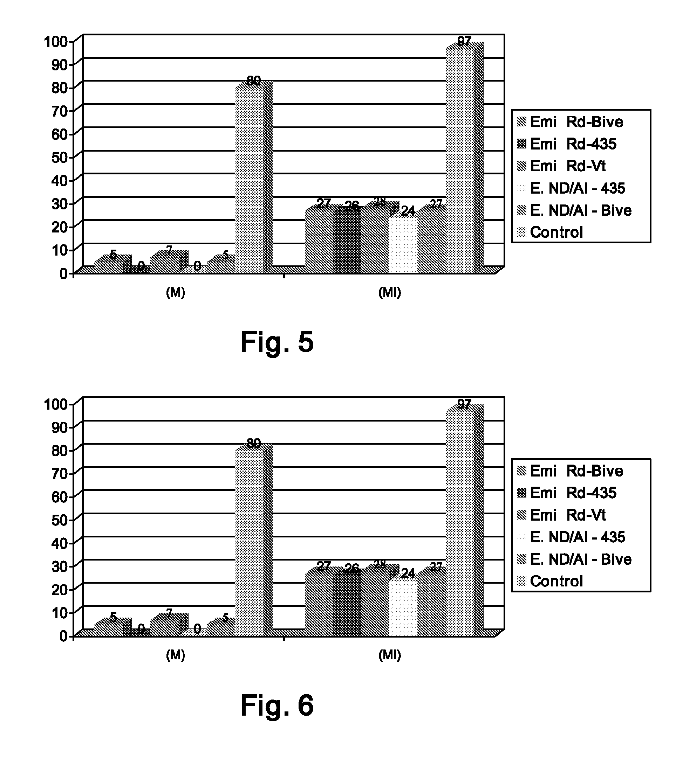

Potency results against VNDV and HPAIV-H5N2 are graphically shown in Figures 5 and 6, respectively.

The results show that all three recombinant inactivated vaccines rNVD/LS-H5 with anchoring of the present invention are capable of providing in commercial broilers having parental immunity to ND and AI viruses, protections equal to or higher than 90% to mortality (M) induced by the VNDV challenge virus (Figure 5). In addition, and regardless the H5-gene with which they were cloned, all three recombinant inactivated vaccines also provided protections equal to or higher than 80% to mortality (M) induced by HPAIV-H5N2, equally to the conventional emulsified vaccines produced with inactivated whole-viruses used to control ND and AI.

The protection results indicate that the recombinant inactivated vaccines rNDV/LS-H5 with anchoring of the present invention, can be successfully used to control HPAI in commercial broilers with parental immunity to AI and ND viruses, with protections similar to those provided by conventional vaccines produced with inactivated whole-virus of AI, but having the further advantage that with the exclusive use of recombinant active and inactive vaccines the biosafety is complete, and the DIVA system can be established allowing the conjoint use of vaccination programs and AI eradication.

## EXAMPLE 6D

In order to determine the anchoring effect in real field conditions, challenges were made using a VNDV and a HPAIV-H5N2 at 21 DPV in groups of commercial broilers with parental immunity to ND and AI, which were immunized as indicated in Table 5, with two emulsified commercial vaccines against avian influenza and Newcastle disease, produced with emulsified inactivated whole-virus called **E. ND/AI-435,** and **E. ND/AI-Bive,** as well as three emulsified inactivated vaccines of the present invention, without anchoring, obtained according to Examples 5D **(Emi-Re-Bive),** 5E **(Emi-Re-435)** and 5F **(Emi-Re-Vt).**

TABLE 5. Potency in commercial broilers having parental immunity to ND and AI, immunized with inactivated vaccines produced with the recombinant virus rNDV/LS-H5 without anchoring (Re)

| Group | No. of chickens | Administered vaccines at 14 days-old by SC route | Number of commercial broilers per challenge strain administered at 21 DPV | |
|---|---|---|---|---|
| | | | HPAIV-H5N2 | VVNDV |
| 1 | 50 | Emi Re-Bive | 25 | 25 |
| 2 | 50 | Emi Re-435 | 25 | 25 |
| 3 | 50 | Emi Re-Vt | 25 | 25 |
| 4 | 50 | E. ND/AI-435 | 25 | 25 |

(continued)

| Group | No. of chickens | Administered vaccines at 14 days-old by SC route | Number of commercial broilers per challenge strain administered at 21 DPV | |
|---|---|---|---|---|
| | | | HPAIV-H5N2 | VVNDV |
| 5 | 50 | E. ND/AI-Bive | 25 | 25 |
| 6 | 50 | Positive control | 25 | 25 |
| 7 | 20 | Negative control | ---- | ---- |

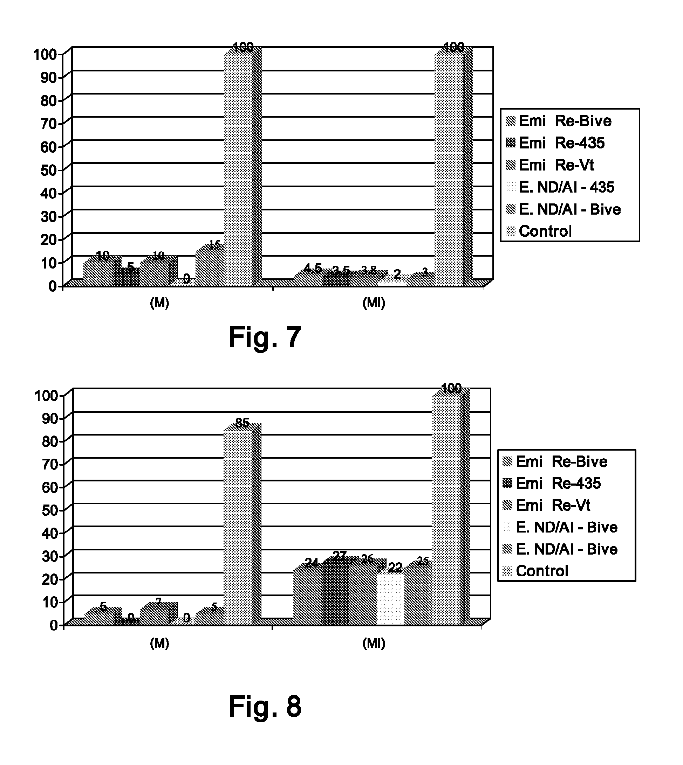

Potency results against VNDV and HPAIV-H5N2 are graphically shown in Figures 7 and 8, respectively.

Unexpectedly, the results show that all three recombinant inactivated vaccines rNVD/LS-H5 without anchoring, are also capable of providing in commercial broilers having parental immunity protections equal to or higher than 90% to mortality (M) induced by the VNDV challenge virus (Figure 7), equally, and regardless of H5-gene with which all three recombinant inactivated vaccines were cloned, all also provided protections equal to or higher than 80% to mortality (M) induced by HPAIV-H5N2, like the recombinant inactivated vaccines rNDV/LS-H5 with anchoring, and conventional emulsified vaccines produced with inactivated whole-virus ND/AI-Bive and ND/AI-435.

These studies corroborate the success of the present invention, since it has been proved that the recombinant vaccines against AI in the inactivated form, using an emulsion or pharmaceutically acceptable vehicle, adjuvant or excipient for its use in susceptible birds, allow an excellent immune response capable of providing protections of 100% to challenges with HPAIV in SPF chicken and higher than 80% in broilers having parental immunity to ND and AI viruses, which is contrary and non-suggested to what was thought about the recombinant virus replication in the immunized bird being essential for the protein of concern to express itself in enough amount to produce a suitable immune response in the bird.

The use of inactivated vaccines is essential to achieve a suitable protection in the field-level to prevent mortality caused by HPAIV and VNDV, since under field conditions in industrial poultry exploitations, the only use of conventional active vaccines against ND, or recombinant active against AI, may not suffice.

Although specific embodiments of the invention have been illustrated and described, it shall be appreciated that several modifications thereto are possible, as may be the AI virus or adenovirus strain used, the emulsion type or the vehicles employed. Therefore, the present invention shall not be construed as limited except for the prior art teachings and for the appended claims.

## Claims

1. A recombinant vaccine comprising a viral vector and a pharmaceutically acceptable vehicle, adjuvant or excipient, **characterized in that** the viral vector is inactivated and has an exogenous nucleotide sequence inserted that codifies for an antigen of a disease of concern.

2. A recombinant vaccine, according to claim 1, further **characterized in that** the exogenous nucleotide sequence codifies for an antigen selected from influenza, infectious laryngotracheitis, infectious bronchitis, bursa of Fabricius' infection (Gumboro), hepatitis, viral rhinotracheitis, infectious coryza, *Mycoplasma hyopneumonieae,* pasteurellosis, Porcine Respiratory and Reproductive Syndrome (PRRS), circovirus, bordetellosis or parainfluenza.

3. A recombinant vaccine, according to claim 2, further **characterized in that** the exogenous nucleotide sequence consists of the gene coding for hemagglutinin (HA) of the avian influenza virus.

4. A recombinant vaccine, according to claim 3, further **characterized in that** the gene coding for hemagglutinin (HA) is selected from at least one of the hemagglutinin (HA) subtypes H1, H2, H3, H5, H6, H7 or H9 of the avian influenza virus.

5. A recombinant vaccine, according to claim 4, further **characterized in that** the gene coding for the hemagglutinin (HA) is the subtype H5.

14

**6.** A recombinant vaccine, according to claim 1, further **characterized in that** the viral vector is selected from adenovirus or paramixovirus.

**7.** A recombinant vaccine, according to claim 6, further **characterized in that** the viral vector is selected from paramixovirus.

**8.** A recombinant vaccine, according to claim 7, further **characterized in that** the paramixovirus is the Newcastle disease virus.

**9.** A recombinant vaccine, according to claim 8, further **characterized in that** the Newcastle disease virus is selected from LaSota, Ulster, QV4, B1, CA 2002, Roakin, Komarov, Clone 30, or VGGA strains, or strains from the Newcastle disease genetic groups I to V.

**10.** A recombinant vaccine, according to claim 6, further **characterized in that** the viral vector is selected from adenovirus.

**11.** A recombinant vaccine, according to claim 10, further **characterized in that** the adenovirus is selected from avian or porcine adenovirus.

**12.** A recombinant vaccine, according to claim 11, further **characterized in that** the adenovirus is an avian adenovirus type 9.

**13.** A recombinant vaccine, according to claim 11, further **characterized in that** the adenovirus is a porcine adenovirus type 5.

**14.** A recombinant vaccine, according to claim 1, further **characterized in that** the pharmaceutically acceptable vehicles for the vaccine are preferably aqueous solutions or emulsions.

**15.** A recombinant vaccine, according to claim 14, further **characterized in that** a water-oil emulsion is used as vehicle.

**16.** A recombinant vaccine, according to claim 1, further **characterized in that** the required titer for the viral vector is similar to that required for a recombinant active-virus vaccine.

**17.** A recombinant vaccine, according to claim 16, further **characterized in that** the virus concentration required to achieve the antigenic response is between $10^2$ and $10^{10}$ D150%/ml.

**18.** A recombinant vaccine, according to claim 7, further **characterized in that** the virus concentration required to achieve the antigenic response is between $10^4$ and $10^{10}$ DIEP50%/ml.

**19.** A recombinant vaccine, according to claim 18, further **characterized in that** the virus concentration is between $10^8$ and $10^9$ DIEP50%/0.5 ml per chicken when the vaccine is prepared to be administered in chickens.

**20.** A recombinant vaccine, according to claim 19, further **characterized in that** the vaccine has $10^{8.5}$ DIEP50%/0.5 ml per chicken.

**21.** A recombinant vaccine, according to claim 10, further **characterized in that** the virus concentration required to achieve the antigenic response is between $10^2$ and $10^8$ DIEP50%/ml.

**22.** A recombinant vaccine, according to claim 1, further **characterized in that** the virus concentration required to achieve the antigenic response is the vaccine is prepared to be subcutaneously or intramuscularly administered.

**23.** A vaccination method against animal's diseases, **characterized in that** it comprises administering to an animal a recombinant vaccine comprising an inactivated viral vector having inserted an exogenous nucleotide sequence coding for an antigen of said disease.

**24.** A vaccination method against animal's diseases, according to claim 23, further **characterized in that** a recombinant vaccine is additionally administered to the animal, comprising an active viral vector identical to the inactivated viral vector, having an exogenous nucleotide sequence inserted coding for an antigen of said disease.

25. An identification method between infected animals and vaccinated animals, useful for the control and eradication of diseases, **characterized in that** it comprises:

a) subjecting to a first antibodies detection method, at least one sample of at least one animal having received a recombinant vaccine of inactivated viral vector having inserted an exogenous nucleotide sequence coding for an antigen of a disease caused by a pathogen, to detect if there are antibodies present in said sample, corresponding to said antigen;

b) subjecting to a second antibodies detection method, at least one sample from the same animal which sample was subjected to the first antibodies detection method, to detect if there are antibodies present in said sample corresponding to the pathogen causing the disease;

c) determining if the animal is infected or vaccinated from the results of the first and second antibodies detection methods.

Fig. 1

Fig. 2

Fig. 3

Fig. 4

Fig. 5

Fig. 6

Fig. 7

Fig. 8

## INTERNATIONAL SEARCH REPORT

| International application No. |
|---|
| PCT/ IB 2008/003150 |

**A. CLASSIFICATION OF SUBJECT MATTER**

*A61K 39/145* (2006.01)

According to International Patent Classification (IPC) or to both national classification and IPC

**B. FIELDS SEARCHED**

Minimum documentation searched (classification system followed by classification symbols)

A61K 39

Documentation searched other than minimum documentation to the extent that such documents are included in the fields searched

Electronic data base consulted during the international search (name of data base and, where practicable, search terms used)

EPODOC,WPI,MEDLINE,BIOSIS,EMBASE

**C. DOCUMENTS CONSIDERED TO BE RELEVANT**

| Category* | Citation of document, with indication, where appropriate, of the relevant passages | Relevant to claim No. |
|---|---|---|
| X | HULSKOTTE E . ET AL., "Chemical inactivation of recombinant vaccinia viruses and the effects on antigenicity and immunogenicity of recombinant simian immunodeficiency virus invelope glycoproteins" Vaccine (1997), vol. 15, n° 17/ 18, pp.1839-1845. the whole document | 1 |
| X | ZAJAC, P. ET AL., "Enhanced generation of cytotoxic T lymphocytes using recombinant vaccinia virus expressing human tumor-associated antigens and B7 costimulatory molecules". Cancer Research (1998), 58, pp.4567-4571. the whole document | 1 |
| A | DataBase WPI, Thomson Scientific, London, GB [retrieved on 18.01.2010]Retrieved from EPOQUE; Número of acceso 2006-729989 & WO 2007025420 (HARBIN VETERINARY MEDICINE INST. CHINESE) 08.03.2007 | 2-25 |

☒ Further documents are listed in the continuation of Box C.   ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | | |
| | | "&" | document member of the same patent family |

| Date of the actual completion of the international search | Date of mailing of the international search report |
|---|---|
| 21 January 2010       (21.01.2010) | **(09/02/2010)** |
| Name and mailing address of the ISA/ O.E.P.M. Paseo de la Castellana, 75 28071 Madrid, España. Facsimile No.  34 91 3495304 | Authorized officer M. Hernández Cuéllar Telephone No. 34 91 349 84 09 |

Form PCT/ISA/210 (second sheet) (July 2009)

INTERNATIONAL SEARCH REPORT

International application No.

PCT/ IB 2008/003150

| Box No. II    Observations where certain claims were found unsearchable (Continuation of item 2 of first sheet) |
| --- |

This international search report has not been established in respect of certain claims under Article 17(2)(a) for the following reasons:

1. ☐  Claims Nos.:
   because they relate to subject matter not required to be searched by this Authority, namely:

2. ☒  Claims Nos.:  **2-25 (all partially)**
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

| Box No. III    Observations where unity of invention is lacking (Continuation of item 3 of first sheet) |
| --- |

This International Searching Authority found multiple inventions in this international application, as follows:

1. ☐  As all required additional search fees were timely paid by the applicant, this international search report covers all searchable claims.

2. ☐  As all searchable claims could be searched without effort justifying additional fees, this Authority did not invite payment of additional fees.

3. ☐  As only some of the required additional search fees were timely paid by the applicant, this international search report covers only those claims for which fees were paid, specifically claims Nos.:

4. ☐  No required additional search fees were timely paid by the applicant. Consequently, this international search report is restricted to the invention first mentioned in the claims; it is covered by claims Nos.:

Remark on Protest          ☐   The additional search fees were accompanied by the applicant's protest and, where applicable, the payment of a protest fee.
                           ☐   The additional search fees were accompanied by the applicant's protest but the applicable protest fee was not paid within the time limit specified in the invitation.
                           ☐   No protest accompanied the payment of additional search fees.

Form PCT/ISA/210 (continuation of first sheet (2)) (April 2005)

## INTERNATIONAL SEARCH REPORT

| | International application No. |
|---|---|
| | PCT/IB 2008/003150 |

**C (continuation).** DOCUMENTS CONSIDERED TO BE RELEVANT

| Category* | Citation of documents, with indication, where appropriate, of the relevant passages | Relevant to claim No. |
|---|---|---|
| A | DataBase WPI, Thomson Scientific, London, GB [retrieved on 18.01.2010]Retrieved from EPOQUE; Número of acceso 2007-344805 & WO 2007128169 (HARBIN VETERINARY MEDICINE INST. CHINESE) 15.11.2007 | 2-25 |
| A | WO 2007104782 A1 (INTERVET INTERNATIONAL B.V.) 20.10.2007,the whole document | 2-25 |
| A | US 20070178115 A1 (TANG et al.) 02.08.2007,the whole document | 2-25 |
| A | CAPUA I. ET AL., "Development of DIVA (Differentiating Infected from vaccinated Animals) strategy using a vaccine containing a heterologous neuraminidase for the control of avian influenza" Avian Pathology (2002), 32, 47-55. the whole document. | 25 |

Form PCT/ISA/210 (continuation of second sheet) (July 2009)

## INTERNATIONAL SEARCH REPORT

Information on patent family members

International application No.

PCT/ IB 2008/003150

| Patent document cited in the search report | Publication date | Patent family member(s) | Publication date |
|---|---|---|---|
| WO 2007025420 A | 08.03.2007 | CN 1772887 A<br>CN 1298845 C | 17.05.2006<br>07.02.2007<br>07.02.2007 |
| WO 2007128169 A | 15.11.2007 | CN 1869234 A<br>CN 100487119 C | 29.11.2006<br>13.05.2009 |
| WO 2007104782 A | 20.09.2007 | CA 2638975 A<br>AU 2007224430 A<br>EP 1996610 A<br>EP 20070726920<br>MX 2008011728 A<br>KR 20090007706 A<br>CN 101421294 A<br>ZA 200807553 A<br>JP 2009529861 T<br>US 2010008945 A | 20.09.2007<br>20.09.2007<br>03.12.2008<br>15.03.2007<br>10.12.2008<br>20.01.2009<br>29.04.2009<br>24.06.2009<br>27.08.2009<br>14.01.2010 |
| US 2007178115 A | 02.08.2007 | WO 2007022151 A<br>CA 2619174 A<br>AU 2006279612 A<br>EP 1924282 A<br>EP 20060813446<br>KR 20080093405 A<br>CN 101365484 A<br>JP 2009512421 T | 22.02.2007<br>22.02.2007<br>22.02.2007<br>28.05.2008<br>15.08.2006<br>21.10.2008<br>11.02.2009<br>26.03.2009 |

Form PCT/ISA/210 (patent family annex) (July 2009)

INTERNATIONAL SEARCH REPORT
Information on patent family members

International application No.

PCT/ IB 2008/003150

**Box III**

**Claims 2-25 (all in part)**

The stated claims do not meet the requirements of clarity, concision and support (PCT Articles 5 and 6). These claims encompass multiple potential vaccines by combining multiple antigens and vectors, whilst the application discloses in line with PCT Articles 5 and 6 only one alternative. Consequently, the search was limited to the vaccine identified by the applicants as rNDV/LS-H5 based on an inactive Newcastle viral recombinant vector that expresses the haemagglutinin H5 of the avian influenza virus.

Form PCT/ISA/210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- Newcastle Disease. OIE Manual of Diagnostic Tests and Vaccines for Terrestrial Animals. Office International des Epizooties, 2008, 576-589
- Avian Influenza. OIE Manual of Diagnostic Test and Vaccines for Terrestrial Animals. Office International des Epizooties, 2008, 465-481
- Vaccines, Vaccination and Immunology for avian influenza viruses in poultry. **Swayne, D ; Kapczynski, D.** Avian Influenza. Blackwell Publishing, 2008, 407-451
- **Capua, I et al.** Development of a DIVA (differentiating infected from vaccinated animals) strategy using a vaccine containing a heterologous neuraminidase for the control of avian influenza. *Avian Pathology,* vol. 32 (1), 47-55
- **Ge ; Deng ; Tian et al.** Newcastle disease virus-based live attenuated vaccine completely protects chickens and mice. *J. Vir.,* vol. 81 (1), 150-158
- **Park ; Man Seong et al.** Engineered viral vaccine constructs with dual specificity: Avian Influenza and Newcastle disease. *PNAS,* 12 May 2006, vol. 103 (21), 8203-8208
- **Soto et al.** Inactivated mexican H5N2 avian influenza vaccine protects chickens from the asiatic highly pathogenic H5N1 avian influenza virus. *Proceedings of the 56th Western Poultry Disease Conference (WPDC). USA,* 2007, 79
- Vaccines, Vaccination and Immunology for avian influenza viruses in poultry. **Swayne, D. ; Kapczynski, D.** Avian Influenza. Blackwell Publishing, 2008, 407-451